# EUROPÄISCHE PATENTANMELDUNG

(11) **EP 1 327 439 A2**
(43) Veröffentlichungstag der Anmeldung: **16.07.2003**
(21) Anmeldenummer: 03000180.4
(22) Anmeldetag: 07.01.2003
(51) Int. Cl.: A61K 7/50

(54) **Dreiphasensysteme**

(30) Priorität: 11.01.2002 DE 10200724
(71) Anmelder: Clariant GmbH, 65929 Frankfurt am Main (DE)
(72) Erfinder: Miller, Dennis, Dr., 65779 Kelkheim (DE); Henning, Torsten, Dr., 65812 Bad Soden (DE)
(74) Vertreter: Mikulecky, Klaus

(57) **Zusammenfassung**

Gegenstand der Erfindung sind Dreiphasensysteme, enthaltend
a) eine Polyethylenglykol-Phase, enthaltend
   a1) 50 - 100 Gew.-% mindestens eines Polyethylenglykols und
   a2) 0 - 50 Gew.-% Wasser,
b) eine Öl-Phase und
c) eine Mikroemulsions-Phase, enthaltend
   c1) die Komponenten der Polyethylenglykol-Phase a),
   c2) die Komponenten der Öl-Phase b) und
   c3) mindestens ein Tensid.

Die Dreiphasensysteme sind thermodynamisch stabil und eignen sich bevorzugt als kosmetische Mittel, besonders bevorzugt als Badeöle.

## Beschreibung

Das Phasenverhalten von Kohlenwasserstoff/Tensid/Wasser-Systemen ist Bestandteil zahlreicher Untersuchungen. Durch geeignete Auswahl der Komponenten lassen sich Dreiphasensysteme erhalten, die aus einer oberen Kohlenwasserstoff-Phase, einer mittleren Mikroemulsions-Phase aus solubilisiertem Kohlenwasserstoff, Tensid und Wasser und einer unteren Wasserphase bestehen. Derartige Dreiphasensysteme werden beschrieben in
- M. Kahlweit und R. Strey, "The Phase behaviour of H₂O-Oil-Nonionic Amphiphile Ternary Systems" in "Microemulsion Systems", Surfactant Science Series Vol. 24, Dekker, New York,1987.
- K. Shinoda and S. Friberg, "Emulsions and Solubilization", Wiley, New York, 1986.
- H.G. Hauthal und K. Quitsch "Neues über Mikroemulsionen", Z. Chem., 30, 274-281 (1990).

In der Anwendung sind derartige Dreiphasensysteme auf technische Anwendungen und Laboranwendungen (Tertiär-Erdölförderung, Medien für chemische Reaktionen etc.) beschränkt. Grund hierfür ist die Toxizität der oberen Kohlenwasserstoff-Phase (Toluol etc.), die einen Einsatz im Kosmetik- und Haushaltsbereich nicht zulässt. Bei Ersatz der Kohlenwasserstoffe durch die in der Kosmetik üblichen Öle (z.B. Mineralöle, Polydecene, Triglyceride, natürliche Öle und Ester) wird die mittlere Mikroemulsions-Phase - und damit das gesamte Dreiphasensystem - thermodynamisch instabil.

Überraschend wurde nun gefunden, dass dreiphasige Mischungen, die anstatt der Wasserphase eine Polyethylenglykol-Phase enthalten, auch dann thermodynamisch stabil sind, wenn die Ölphase aus den in der Kosmetik gebräuchlichen Ölen, wie z.B. Mineralölen, Polydecenen, Triglyceriden, natürlichen Ölen und Estern, besteht. Bei der Polyethylenglykol-Phase kann es sich um eine Polyethylenglykol/Wasser-Mischung oder um reine Polyethylenglykole handeln.

Das Vorliegen dreier Phasen verleiht den erfindungsgemäßen Dreiphasensystemen ein sehr ästhetisches Aussehen. Durch Zugabe von Farbstoffen, besonders durch Zugabe verschiedenfarbiger wasserlöslicher und öllöslicher Farbstoffe, kann das besondere Erscheinungsbild noch weiter herausgestellt werden. Die Verwendung ist nicht auf den Kosmetik- und Haushaltsbereich beschränkt. Ebenfalls möglich ist die Verwendung im technischen Bereich, im Laborbereich, aber auch als Spielzeug, Werbeträger, Kunstgegenstand oder auch als Unterrichtsmittel zur Demonstration physikalischer Phänomene.

Gegenstand der Erfindung sind Dreiphasensysteme, enthaltend
a) eine Polyethylenglykol-Phase, enthaltend
   a1) 50 - 100 Gew.-% mindestens eines Polyethylenglykols und
   a2) 0 - 50 Gew.-% Wasser,
b) eine Öl-Phase und
c) eine Mikroemulsions-Phase, enthaltend
   c1) die Komponenten der Polyethylenglykol-Phase a),
   c2) die Komponenten der Öl-Phase b) und
   c3) mindestens ein Tensid.

Bevorzugt sind Dreiphasensysteme, die
a) 10 bis 80 Gew.-%, bevorzugt 10 bis 50 Gew.-%, der Polyethylenglykol-Phase,
b) 10 bis 80 Gew.-%, bevorzugt 10 bis 50 Gew.-%, der Öl-Phase und
c) 10 bis 80 Gew.-%, bevorzugt 10 bis 50 Gew.-%, der Mikroemulsions-Phase enthalten.

Bevorzugt sind Polyethylenglykol-Phasen a) die 50 bis 100 Gew.-%, bevorzugt 75 bis 100 Gew.-%, besonders bevorzugt 85 bis 100 Gew.-%, Polyethylenglykol und 0 bis 50 Gew.-%, bevorzugt 0 bis 25 Gew.-%, besonders bevorzugt 0 bis 15 Gew.-%, Wasser enthalten.

In einer besonderen Ausführungsform enthält die Polyethylenglykol-Phase a) 100 Gew.-% Polyethylenglykol.

Die Polyethylenglykol-Phase a) kann ein oder mehrere Polyethylenglykole enthalten.

Die Polyethylenglykole besitzen bevorzugt ein Molekulargewicht von 150 bis 35 000 g/mol, bevorzugt 200 bis 800 g/mol.

Bei den Tensiden der Mikroemulsions-Phase c) kann es sich um nichtionische, kationische, anionische und/oder amphotere Tenside handeln.

Bei den nichtionischen Tensiden handelt es sich bevorzugt um Fettalkoholethoxylate, Dimethylaminoxide, ethoxylierte Rizinusöle, Alkylpolyglucoside, Fettsäuresorbitolester, Fettsäurepolyglycerolester, ethoxylierte Fettsäurepolyglycerolester, Fettsäuremonoethanolamidethoxylate, Glycerinmonound -diestern von Fettsäuren und/oder Phosphorsäuretriester.

Ebenfalls bevorzugt als nichtionische Tenside sind (C₈-C₂₂)-Alkyl- oder Alkenylethoxylate mit 2 bis 20 Ethylenoxidgruppen.

Bei den anionischen Tensiden handelt es sich bevorzugt um Phosphorsäuremonoester, Phosphorsäurediester, Alkylsulfate, Alkylethersulfate, bevorzugt Natriumlaurethsulfat, Alkylamidopolyglykolethersulfate, Alkylpolyglykolethercarboxyate, Alkylpolyglykolethersulphosuccinate und/oder Fettsäureisethionate.

Bei den amphoteren Tensiden handelt es sich bevorzugt um Acylglutamate, Alkylamidopropylbetaine, bevorzugt Cocoamidopropylbetain, Fettsäuremethyltauride, Fettsäuresarcoside und/oder Amphoacetate.

In einer besonderen Ausführungsform handelt es sich bei den Tensiden um Betaine, Alkylethersulfate oder Mischungen derselben.

Bevorzugt enthalten die Dreiphasensysteme 1 bis 20 Gew.-%, besonders bevorzugt 5 bis 20 Gew.-%, Tenside.

Als Öl-Phase eignen sich bevorzugt Mineralöle, Polydecene, Triglyceride, z.B. Capric/Caprylic Triglyceride, natürliche Öle, z.B. Orangenöl, und/oder Ester, bevorzugt Stearate, Palmitate und Myristate.

Als Dreiphasensysteme bevorzugt sind solche, bei denen die Komponenten der Ölphase b) in der Mikroemulsions-Phase c) mit einem Solubilisierungsgrad S von größer oder gleich 0.8, bevorzugt größer oder gleich 1.5, solubilisiert sind. Beim Solubilisierungsgrad S handelt es sich um das Volumenverhältnis der Öl-Komponenten zu den Tensid-Komponenten.

In einer besonderen Ausführungsform enthalten die Dreiphasensysteme noch zusätzlich polare organische Verbindungen, bevorzugt Hydroxyverbindungen und/oder Polyhydroxyverbindungen, besonders bevorzugt Glycerin, Propylenglykol, Ethanol, Hexylenglykol und/oder Isopropanol.

In einer besonderen Ausführungsform enthalten die Dreiphasensystem noch wasserlösliche und/oder öllösliche, bevorzugt verschiedenfarbige, Farbstoffe. Hierdurch lassen sich vorteilhafte ästhetische Effekte erzielen.

Bevorzugt handelt es sich bei den Dreiphasensystemen um kosmetische Mittel. Besonders bevorzugt sind Badeöle. Vorteilhafterweise werden die Badeöle vor dem Gebrauch geschüttelt, so dass sie als Emulsion zum Badewasser gegeben werden. Als zusätzliche Inhaltsstoffe können die in der Kosmetik gängigen Stoffe, wie z.B. Parfümöle, ätherische Öle, Pflanzenextrakte, Farbmittel, kationische Polymere, Solubilisierhilfsmittel, Vitamine und Vitaminderivate, perlglanzgebende Mittel, Konservierungsstoffe, Hautgefühlsverbesserer, Stabilisierungsmittel, UV-Absorber, Hydroxysäuren und deren Salze, verwendet werden.

Aufgrund der thermodynamischen Stabilität der erfindungsgemäßen Dreiphasensysteme kann deren Herstellung vorteilhafterweise durch einfaches Mischen der einzelnen Komponenten der Phasen a), b) und c) erfolgen.

Das thermodynamische Gleichgewicht der drei Phasen stellt sich von alleine ein.
In einer bevorzugten Ausführungsform werden die hydrophilen Komponenten (Polyethylenglykole, Wasser, wasserlösliche Farbstoffe etc.) einschließlich der Tenside miteinander vermischt. Separat davon werden die hydrophoben, wasserunlöslichen Komponenten (Öle, öllösliche Farbstoffe etc.) miteinander vermischt. Anschließend werden die hydrophile Mischung und die hydrophobe Mischung miteinander vermischt.

Gegenstand der Erfindung sind demnach auch Dreiphasensysteme, erhältlich durch Herstellen einer Mischung, enthaltend
i) Polyethylenglykol(e),
ii) gegebenenfalls Wasser,
iii) Öl-Komponente(n) und
iv) Tensid(e).

Bevorzugt sind Dreiphasensysteme, erhältlich durch Herstellen einer Mischung, enthaltend
i) 10 bis 70 Gew.-%, bevorzugt 25 bis 60 Gew.-%, Polyethylenglykol(e),
ii) 0 bis 70 Gew.-%, bevorzugt 0 bis 20 Gew.-%, Wasser,
iii) 10 bis 70 Gew.-%, bevorzugt 25 bis 60 Gew.-%, Öl-Komponente(n) und
iv) 2 bis 20 Gew.-%, bevorzugt 5 bis 20 Gew.-% Tensid(e).

In einer besonderen Ausführungsform sind die Dreiphasensysteme frei von Wasser. In einer bevorzugten Ausführungsform enthalten die Mischungen zusätzlich wasserlösliche und/oder öllösliche Farbstoffe.
Bei den bevorzugten Polyethylenglykolen, Öl-Komponenten und Tensiden handelt es sich um die vorab schon beschriebenen Verbindungen.

### Beispiele

In den Beispielen wurde der Ethoxylierungsgrad der Tenside so optimiert, dass ungefähr gleiche Mengen von Ölphase und hydrophiler Phase in der Mikroemulsion solubilisiert wurden. S ist der Grad der Solubilisierung des Öls auf Basis des Volumenverhältnisses. Oleth-8 und Oleth-10 sind die INCI-Bezeichnungen für Oleylalkoholpolyglykolether mit 8 bzw. 10 Mol Ethylenoxid. PEG-8 ist die INCI-Bezeichnung für Polyethylenglykol mit einer mittlerer Molmasse von 400 g/mol.
Bei den Mengenangaben handelt es sich um Gew.-%.

### Beispiele 1 bis 3:

| Bsp. | Polyethylenglykol-Phase | | Tensid | | Ölphase | Phasenvolumen | | | S |
|---|---|---|---|---|---|---|---|---|---|
| | PEG-8 | Wasser | Oleth-8 | Oleth-10 | Mineralöl | Unten | Mitte | Oben | |
| 1 | 43,1 | 10,8 | 6,9 | 0 | 39,2 | 28,9 | 34,8 | 36,3 | 1,5 |
| 2 | 27,2 | 6,8 | 17,3 | 0 | 48,7 | 14,0 | 82,0 | 4,0 | 2,2 |
| 3 | 36,9 | 15,8 | 9,0 | 0,2 | 38,1 | 15,5 | 62,5 | 22,0 | 2,5 |

### Beispiel 4: Dreiphasen-Badeöl

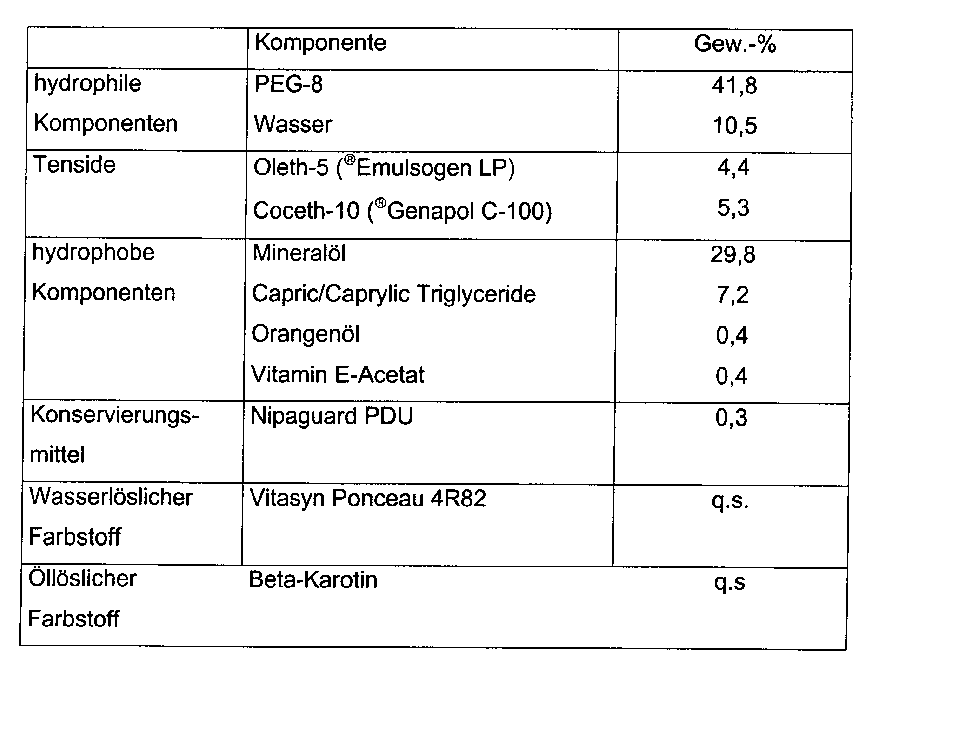

Das Badeöl separierte in folgende drei Phasen:

| | |
|---|---|
| obere Phase (rot) | 28,5 Vol.-% |
| mittlere Phase (orange) | 48,0 Vol.-% |
| untere Phase (gelb) | 23,5 Vol.-% |

Die Herstellung der Beispiele 1 bis 4 erfolgte so, dass eine Mischung der hydrophilen Komponenten und separat davon eine Mischung der hydrophoben Komponenten hergestellt wurde. Anschließend wurden die Tenside zur hydrophilen Mischung gegeben. Zuletzt wurden die hydrophile Mischung und die hydrophobe Mischung miteinander vermischt.

## Patentansprüche

1. Dreiphasensystem, enthaltend
a) eine Polyethylenglykol-Phase, enthaltend
a1) 50 - 100 Gew.-% mindestens eines Polyethylenglykols und
a2) 0 - 50 Gew.-% Wasser,
b) eine Öl-Phase und
c) eine Mikroemulsions-Phase, enthaltend
c1) die Komponenten der Polyethylenglykol-Phase a),
c2) die Komponenten der Öl-Phase b) und
c3) mindestens ein Tensid.

2. Dreiphasensystem nach Anspruch 1, **dadurch gekennzeichnet, dass** es
a) 10 bis 80 Gew.-%, bevorzugt 10 bis 50 Gew.-%, der Polyethylenglykol-Phase,
b) 10 bis 80 Gew.-%, bevorzugt 10 bis 50 Gew.-%, der Öl-Phase und
c) 10 bis 80 Gew.-%, bevorzugt 10 bis 50 Gew.-%, der Mikroemulsions-Phase enthält.

3. Dreiphasensystem nach Anspruch 1 und/oder 2, **dadurch gekennzeichnet, dass** die Polyethylenglykol-Phase a) 50 bis 100 Gew.-%, bevorzugt 75 bis 100 Gew.-%, besonders bevorzugt 85 bis 100 Gew.-%, Polyethylenglykol und 0 bis 50 Gew.-%, bevorzugt 0 bis 25 Gew.-%, besonders bevorzugt 0 bis 15 Gew.-%, Wasser enthält.

4. Dreiphasensystem nach Anspruch 3, **dadurch gekennzeichnet, dass** die Polyethylenglykol-Phase a) 100 Gew.-% Polyethylenglykol enthält.

5. Dreiphasensystem nach mindestens einem der Ansprüche 1 bis 4,
**dadurch gekennzeichnet, dass** die Polyethylenglykole der Polyethylenglykol-Phase
a) ein Molekulargewicht von 150 bis 35 000 g/mol, bevorzugt 200 bis 800 g/mol, besitzen.

6. Dreiphasensystem nach mindestens einem der Ansprüche 1 bis 5, **dadurch gekennzeichnet, dass** es sich bei den Tensiden der Mikroemulsions-Phase c) um nichtionische, kationische, anionische und/oder amphotere Tenside handelt.

7. Dreiphasensystem nach Anspruch 6, **dadurch gekennzeichnet, dass** es sich bei den nichtionischen Tensiden um Fettalkoholethoxylate, Dimethylaminoxide, ethoxylierte Rizinusöle, Alkylpolyglucoside, Fettsäuresorbitolester, Fettsäurepolyglycerolester, ethoxylierte Fettsäurepolyglycerolester, Fettsäuremonoethanolamidethoxylate, Glycerinmono- und -diestern von Fettsäuren und/oder Phosphorsäuretriester handelt.

8. Dreiphasensystem nach Anspruch 6, **dadurch gekennzeichnet, dass** es sich bei den nichtionischen Tensiden um (C₈-C₂₂)-Alkyl- oder Alkenylethoxylate mit 2 bis 20 Ethylenoxidgruppen handelt.

9. Dreiphasensystem nach Anspruch 6, **dadurch gekennzeichnet, dass** es sich bei den anionischen Tensiden um Phosphorsäuremonoester, Phosphorsäurediester, Alkylsulfate, Alkylethersulfate, bevorzugt Natriumlaurethsulfat, Alkylamidopolyglykolethersulfate, Alkylpolyglykolethercarboxyate, Alkylpolyglykolethersulphosuccinate und/oder Fettsäureisethionate handelt.

10. Dreiphasensystem nach Anspruch 6, **dadurch gekennzeichnet, dass** es sich bei den amphoteren Tensiden um Acylglutamate, Alkylamidopropylbetaine, bevorzugt Cocoamidopropylbetain, Fettsäuremethyltauride, Fettsäuresarcoside und/oder Amphoacetate handelt.

11. Dreiphasensystem nach Anspruch 6, **dadurch gekennzeichnet, dass** es sich bei den Tensiden um Betaine, Alkylethersulfate oder Mischungen derselben handelt.

12. Dreiphasensystem nach mindestens einem der Ansprüche 1 bis 11, **dadurch gekennzeichnet, dass** es sich bei den Komponenten der Öl-Phase b) um Mineralöle, Polydecene, Triglyceride, z.B. Capric/Caprylic Triglyceride, natürliche Öle, z.B. Orangenöl und/oder Ester, bevorzugt Stearate, Palmitate und Myristate, handelt.

13. Dreiphasensystem nach mindestens einem der Ansprüche 1 bis 12, **dadurch gekennzeichnet, dass** die Komponenten der Öl-Phase b) in der Mikroemulsions-Phase c) mit einem Solubilisierungsgrad S von größer oder gleich 0,8, bevorzugt größer oder gleich 1,5, solubilisiert sind.

14. Dreiphasensystem nach mindestens einem der Ansprüche 1 bis 13, **dadurch gekennzeichnet, dass** sie polare organische Verbindungen, bevorzugt Hydroxyverbindungen und/oder Polyhydroxyverbindungen, besonders bevorzugt Glycerin, Propylenglykol, Ethanol, Hexylenglykol und/oder Isopropanol enthält.

15. Dreiphasensystem nach mindestens einem der Ansprüche 1 bis 14, **dadurch gekennzeichnet, dass** sie mindestens einen wasserlöslichen Farbstoff enthält.

16. Dreiphasensystem nach mindestens einem der Ansprüche 1 bis 14, **dadurch gekennzeichnet, dass** sie mindestens einen öllöslichen Farbstoff enthält.

17. Dreiphasensystem nach mindestens einem der Ansprüche 1 bis 14, **dadurch gekennzeichnet, dass** sie mindestens einen wasserlöslichen Farbstoff und mindestens einen öllöslichen Farbstoff enthält.

18. Dreiphasensystem nach Anspruch 17, **dadurch gekennzeichnet, dass** die wasserlöslichen und die öllöslichen Farbstoffe verschiedenfarbig sind.

19. Dreiphasensystem nach mindestens einem der Ansprüche 1 bis 18, **dadurch gekennzeichnet, dass** es sich dabei um ein kosmetisches Mittel handelt.

20. Dreiphasensystem nach Anspruch 19, **dadurch gekennzeichnet, dass** es sich dabei um ein Badeöl handelt.

21. Dreiphasensystem, erhältlich durch Herstellen einer Mischung, enthaltend
i) Polyethylenglykol(e)
ii) gegebenenfalls Wasser
iii) Öl-Komponente(n) und
iv) Tensid(e).

22. Dreiphasensystem nach Anspruch 21, erhältlich durch Herstellen einer Mischung, enthaltend
i) 10 bis 70 Gew.-%, bevorzugt 25 bis 60 Gew.-%, Polyethylenglykol(e),
ii) 0 bis 70 Gew.-%, bevorzugt 0 bis 20 Gew.-%, Wasser,
iii) 10 bis 70 Gew.-%, bevorzugt 25 bis 60 Gew.-%, Öl-Komponente(n) und
iv) 2 bis 20 Gew.-%, bevorzugt 5 bis 20 Gew.-% Tensid(e).

23. Dreiphasensystem nach Anspruch 21 und/oder 22, **dadurch gekennzeichnet, dass** die Polyethylenglykole i) ein Molekulargewicht von 150 bis 35 000 g/mol, bevorzugt 200 bis 800 g/mol, besitzen.

24. Dreiphasensystem nach mindestens einem der Ansprüche 21 bis 23 **dadurch gekennzeichnet, dass** es sich bei der Öl-Komponente iii) um Mineralöle, Polydecene, Triglyceride, z.B. Capric/Caprylic Triglyceride, natürliche Öle, z.B. Orangenöl und/oder Ester, bevorzugt Stearate, Palmitate und Myristate, handelt.

25. Dreiphasensystem nach mindestens einem der Ansprüche 21 bis 24, **dadurch gekennzeichnet, dass** es zusätzlich wasserlösliche und/oder öllösliche, bevorzugt verschiedenfarbige, Farbstoffe enthält.

26. Dreiphasensysteme nach mindestens einem der Ansprüche 21 bis 25, **dadurch gekennzeichnet, dass** es sich dabei um ein kosmetisches Mittel, bevorzugt ein Badeöl, handelt.
